# EUROPEAN PATENT SPECIFICATION

(11) **EP 3 514 131 B1**
(45) Date of publication and mention of the grant of the patent: **08.06.2022**
(21) Application number: 18382024.0
(22) Date of filing: 18.01.2018
(51) Int. Cl.: C05G 5/30

(54) **ANTICAKING COMPOSITIONS FOR SOLID FERTILIZERS, COMPRISING ESTERAMINE COMPOUNDS**
ANTIKLUMPZUSAMMENSETZUNG FÜR FESTE DÜNGEMITTEL MIT ESTERAMINVERBINDUNGEN
COMPOSITIONS D'ENGRAIS SOLIDES ANTIAGGLOMÉRANT COMPRENANT DES COMPOSÉS D'ESTERAMINE

(43) Date of publication of application: 24.07.2019
(73) Proprietor: KAO CORPORATION, S.A., 08210 Barberà del Vallès, Barcelona (ES)
(72) Inventor: Riaza Martinez, Joan Antoni, 08210 Barberà del Vallès (ES); Mundo Blanch, Miquel, 08210 Barberà del Vallès (ES)
(74) Representative: Hoffmann Eitle

(56) References cited:
- EP-A1- 0 711 739
- EP-A1- 2 899 178
- EP-A1- 3 120 922

## Description

### FIELD OF THE INVENTION

The present invention discloses compositions comprising a mixture of esteramine compounds and a solvent for preventing caking in several types of solid fertilizers.

### STATE OF THE ART

Fertilizers are organic or inorganic materials of natural or synthetic origin (other than liming materials) that, when added to a soil, enrich that soil in substances which constitute essential nutrients to the growth of plants.

Fertilizers can be liquid or solid, being alternatively used depending on the purpose. Liquid fertilizers provide fast and direct nourishment for the plants, while solid fertilizers nourish the soil micro organisms, acting therefore as slow releasers of the nutrients.

Among solid fertilizers, the most generally used types are granular simple fertilizers and granular complex fertilizers.

Granular simple fertilizers include primary macronutrients, potassium, phosphorous or nitrogen compounds assimilable by plants. Some examples are potassium chloride (KCI), ammonium nitrate (AN), and calcium nitrate (CN), mono- and diammonium phosphates and urea-based fertilizers.

Granular complex fertilizers include combinations of more than one primary macronutrient. They are usually called NPK and can be differentiated from one another mainly because of their nitrogen, phosphorous and potassium content, expressed as the percentage of nitrogen (N), phosphoric anhydride (P₂O₅) and potassium oxide (K₂O) that the complex fertilizer contains.

Solid fertilizers have as a main application problem that rather frequently, unless treated to avoid it, they experiment caking phenomenon during storage. The recently manufactured solid fertilizer shows good flowability and homogeneity, but the salts therein contained may cause the agglomeration of particles during storage and handling in bulk. Such agglomeration is very much dependent on the temperature, humidity and pressure conditions. If not avoided, the agglomeration of the fertilizer results into the formation of large, agglomerated fertilizer lumps. The bigger the lumps, the more difficult to mix the fertilizer with the soil, causing the loss of the fertilizer efficiency. The agglomeration may further cause the formation of a crust on the surface of the fertilizer. Thus, the caking and crust formation of fertilizers is a major drawback when dosing and applying them, and it also has an impact in economic losses.

Another problem observed during the handling of granular fertilizers (also known as particulate fertilizers) is dust formation during the manufacture, the storage and the transport of the fertilizer particles. Dust formation is mainly caused by the mechanical abrasion resulting from the movements of the fertilizer particles. Other processes that contribute to dust formation are the existence of certain continuous chemical reactions that result in the particle disintegration and the occurrence of a curing process after the initial formation of the particle. Besides, environmental temperature and humidity are factors that further determine the degree of dust formation.

Dust formation can be really a major problem. The dust formed during the handling of the fertilizer particles can remain suspended in the air for a substantially long period of time. The presence of dust in the air can lead to safety, health, environmental and economic problems.

One of the most common solutions used to reduce both the caking problem and the dust formation problem is the addition to the fertilizer of an anticaking additive. Said additive can be applied to the fertilizer just recently manufactured or after a curing period in the warehouse. The fertilizer is treated with a fluid composition comprising the active anticaking compound in order a protective coating comprising said additive is formed over the fertilizer particles or granules surface; the coating quickly solidifies or acquires sufficient viscosity in contact with the solid fertilizer, and provides protection from the caking and dusting disadvantages.

Also in the field of fertilizers industry, as in any other industry nowadays, there exists an increasing demand of more environmentally friendly and more easily biodegradable products, and this applies also for fertilizer anticaking additives. In this regard, the use of surface active agents with reduced eco-toxicity and lower bioaccumulation potential to prevent environmental issues is of particular interest. Such a trend is reflected also by the changes in the regulations affecting this field. For instance, legislation in Europe is being more severe as for the use of fertilizer anticaking additives with low ecological profile in order to ensure that they do not become an environmental risk.

Therefore there is a need for additives that meet environmental standards and that give good results in caking prevention for solid fertilizer particles.

There exist different types of products that can be used as environmentally friendly anticaking fertilizer additives. Among the approaches already described in the prior art there can be mentioned: i) residual flows of natural origin, preferably vegetable flows such as corn and wheat residues (WO2006091076), ii) polymer compounds derived from the bottom fraction of the biodiesel distillation process, wherein said polymers are based on unsaturated monomers containing fatty alkyl groups (WO20091513165), iii) biomass solid particles mixed with a dispersant, being an oil, fat or wax (WO2009074679).

One of the less exploited and barely reported in the state of the art anticaking fertilizer additives are esteramine compounds.

EP0711739 describes mixtures of N-alkyl-N-alkanolamine fatty acid esters and synthetic and/or natural oils as means for the production of non-dusting and non-caking fertilizers. The mixtures can comprise optionally additionally or optionally instead of synthetic and/or natural oils fatty acid compounds (fatty acids or fatty acid esters). Such mixtures present better biodegradability so that their accumulation in agricultural soils can be avoided. N-alkyl-N-alkanolamine fatty acid esters are present in the mixtures in quantity up to 50% by weight, preferably from 10 to 40% by weight. Examples of fertilizers that can be treated with the mixtures of the invention are ammonium nitrate, calcium nitrate, urea, potassium chloride, NPK fertilizers, etc.

EP3120922 describes anticaking compositions for solid fertilizers comprising mixtures of one or more quaternary mono-, di- or tri-ester ammonium compounds and a solvent. The compositions additionally can comprise a mixture of one or more mono-, di- or tri-esteramine compounds, wherein the same is obtained due to the partial quaternization of the esterification reaction products or is added intentionally to the mixture after the quaternization process. The mixture of one or more mono-, di- or tri-esteramine compounds is contained in the compositions in an amount, expressed as percentage by weight, of up to 15%. Similar compositions are known from EP2899178.

From the state of the art set forth above, it can be seen that prevention of caking of solid fertilizers is a complex problem and is far from being considered completely solved. It can also be seen that several approaches with ecofriendly products have been described already. However, there is still a need for ecofriendly anticaking compositions suitable for fertilizers. These ecofriendly compositions shall at the same time have low enough price and solve the different technical problems related to its application: exhibit good anticaking performance, be stable, and have suitable rheologic behaviour with temperature.

### SUMMARY OF THE INVENTION

The first object of the present invention is a composition for preventing the caking of solid fertilizers, said composition comprising a component (a), which comprises a mixture of one or more mono-esteramine, di-esteramine and/or tri-esteramine compounds of formula (111), (II2), and (II3), respectively, and a component (b), which comprises a solvent.

Another object of the present invention is use of a composition according to the invention for preventing the caking of solid fertilizers.

A further object of the present invention is a method for obtaining a composition according to the invention for preventing the caking of solid fertilizers.

A solid fertilizer, characterized in that it has been treated with a composition according to the invention for preventing caking, is also part of the invention.

Another object of the present invention is a method for obtaining a solid fertilizer resistant to caking, characterized in that the solid fertilizer particles are coated or are mixed with a composition according to the invention.

### DETAILED DESCRIPTION OF THE INVENTION

The main object of the present invention is a composition for preventing the caking of solid fertilizers, said composition comprising a component (a), which comprises a mixture of one or more mono-esteramine, di-esteramine and/or tri-esteramine compounds of formula (111), (II2), and (II3), respectively, wherein the sum weight of di-esteramine and tri-esteramine compounds ((II2) and (II3)) is from 70 to 100% wt., based on the total weight of esterified species comprised in component (a); and a component (b), which comprises a solvent, wherein the solvent is selected from mineral oils, paraffins and waxes derived from petroleum and vegetal and animal fats and oils.

### Component (a): Esteramine compounds

The present invention comprises a component (a) that comprises a mixture of one or more mono-esteramine, di-esteramine and/or tri-esteramine compounds of formula (111), (II2), and (II3), respectively: 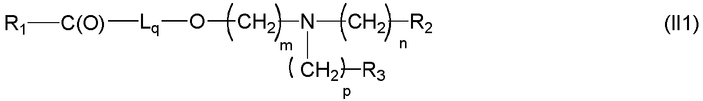 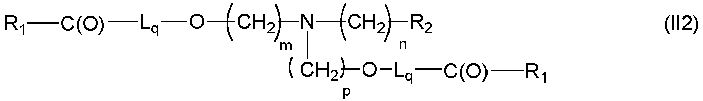 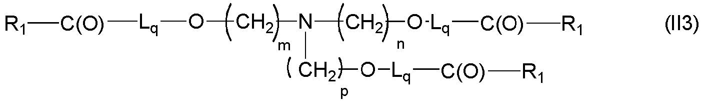 wherein in formulae 111, II2 and II3
R₁ is a linear or branched alkyl containing 5 to 23 carbon atoms and/or a linear alkenyl group containing 5 to 23 carbon atoms and from 1 to 3 double bonds. In formulae 111, II2 and II3 each R₁ can independently represent the same or different linear or branched alkyl chain;
R₂ and R₃ each independently represent -H or-OH,
L represents a -(OCH₂CH₂)ₐ-(OCHR₄CH₂)_{b}- group, wherein R₄ represents an alkyl group containing 1 to 4 carbon atoms, a represents a number within the range of 0 to 20, b represents a number within the range of 0 to 6, and the sum of a+b represents the average alcoxylation degree which corresponds to a number from 0 to 26;
m, n, p each independently represent a number within the range from 1 to 4, and q represents a number with the range from 0 to 26;
wherein in said component (a) the sum weight percentage of di-esteramine and tri-esteramine compound ((112) and (113)) is from 70 to 100% wt., based on the total weight of esterified species comprised in component (a).

The sum weight percentage of di-esteramine and tri-esteramine compounds is calculated with respect to the total weight of esterified species comprised in component (a). Esterified species of component (a) are mono-esteramine, di-esteramine, or tri-esteramine compounds.

The esteramine compounds of the invention can be ethoxylated and/or propoxylated, since a and b can be larger than 0. The order of sequence of the ethylene oxide and propylene oxide groups is not critical for the invention.

In case q is 2 or larger, each L group may be the same or different. Also the (L)q groups contained in the different branches within the compounds of formula (111), (II2), and (II3) may independently represent different meanings.

The sum of a+b preferably represents the average alcoxylation degree which corresponds to a number from 0 to 10, more preferably from 0 to 6, most preferred is 0.

Within the present patent application, when a numerical range is indicated, all the individual numbers included in said range are intended to be included. For example, a range from 0 to 10 will include all the individual numbers 0,1,2,3,4,5,6,7,8,9 and 10. The same shall apply to any other range indicated.

The sum weight percentage of di-esteramine compound represented by formula (II2) and tri-esteramine compound represented by formula (II3) is from 70 to 100% wt., based on the total weight of esterified species comprised in component (a) as defined above.

In the case the weight percentage of di-esteramine is 0, the sum weight percentage of di-esteramine and tri- esteramine corresponds to the weight percentage of tri-esteramine compound.

The sum weight percentage of esterified amine compounds of formula (II2) and (II3) can be determined according to standard NMR methods known by the person skilled in the art.

In a particularly preferred embodiment, component (a) comprises a mixture of one or more mono-esteramine compound of formula (111), di-esteramine compound of formula (II2), and/or tri-esteramine compound of formula (II3), wherein m=n=p=2; R₁-C(O)- is a linear acyl group wherein R₁ is a linear alkyl or a linear alkenyl containing from 11 to 21 carbon atoms, preferably derived from hydrogenated or non-hydrogenated tallow fatty acid; R₂ and R₃ each represent -OH; q is 0 (i.e. the compound is not alkoxylated). The sum weight percentage of di-esteramine compound of formula (II2) and tri-esteramine compound of formula (II3) corresponds to the weight percentage of tri-esteramine compound of formula (II3) and it is 100% wt., based on the total weight of esterified species comprised in component (a). Such a compound may be produced by esterifying tallow fatty acid and triethanolamine.

In another embodiment of the present invention, component (a) comprises a mixture of one or more mono-esteramine, di-esteramine and/or tri-esteramine compounds represented respectively by formulae (111), (II2), and (II3) as defined above, wherein R₂ and R₃ independently represent -OH; m, n and p each represent number 2. The rest of variables have the meanings as indicated above for formulae (111), (II2), and (II3).

In another embodiment of the present invention, component (a) comprises a mixture of one or more mono-esteramine, di-esteramine and/or tri-esteramine compounds represented respectively by formulae (111), (II2), and (II3) as defined above, wherein R₂ represents -H, R₃ represents -OH, m and p each represent number 2, and n represents number 1. The rest of variables have the meanings as indicated above for formulae (111), (II2), and (II3).

In another embodiment of the present invention, component (a) comprises a mixture of one or more mono-esteramine, di-esteramine and/or tri-esteramine compounds represented respectively by formulae (111), (II2), and (II3) as defined above, wherein R₂ and R₃ represent -H, m represents number 2, and n and p each represent number 1. The rest of variables have the meanings as indicated above for formulae (111), (II2), and (II3).

In another embodiment of the present invention, R₁ is a linear or branched alkyl containing 11 to 21 carbon atoms and/or a linear alkenyl group containing 11 to 21 carbon atoms and from 1 to 3 double bonds.

As used herein, the term "alkyl" refers to a straight or branched hydrocarbon chain containing from 5 to 23 carbon atoms.

As used herein, the term "alkenyl" refers to a linear hydrocarbon chain containing from 5 to 23 carbon atoms and from 1 to 3 unsaturations, such as from 1 to 3 double bonds.

Examples of a linear or branched alkyl or a linear alkenyl groups are alkyls or alkenyls derived from oils and fats from plants and animals, such as palm, coconut, sunflower, soybean, palm olein, olive, canola, tall oil or tallow, possibly totally or partially hydrogenated and purified, or synthetic fatty acids such as palmitoleic acid, oleic acid, elaidinic acid, petroselinic acid, linoleic acid, linolenic acid, stearic acid, myristic acid, gadoleic acid, behenic acid and erucic acid or mixtures thereof. Preferably palm oil, coconut oil, tallow fatty acid and hydrogenated tallow fatty acid are used, more preferably tallow fatty acid and hydrogenated tallow fatty acid are used.

"Alkoxy" refers to an alkyl group as defined above bonded to an oxygen atom (R-O-).

### PREPARATION OF COMPONENT (a)

The component (a) comprises a mixture of one or more mono-esteramine, di-esteramine or tri-esteramine compounds according to the invention. This mixture can be prepared by esterification, reacting a fatty acid or a mixture of fatty acids (for example, but not limited to, oleic acid, palm oil, tallow, possibly totally or partially hydrogenated) with an alkanolamine or a mixture of alkanolamines (for example, but not limited to, triethanolamine, methyldiethanolamine or dimethylethanolamine) to obtain a mixture containing esteramine compounds.

### Esterification conditions:

The reaction between the fatty acid and the alkanolamine is an esterification, and it may be conducted in a known way, as described for example in the document ES-A-2021900. Preferably the esterification reaction is carried out at a temperature between 120°C and 220°C, for a period of 2-10 hours, preferably at a reduced pressure of about 5 to 200 mbar and in the presence of one of the catalysts known for the esterification, such as hypophosphorous acid or paratoluenesulfonic acid, and also in the presence of any of the usual stabilizers and antioxidants such as tocopherols, BHT, BHA, etc.

The ratio of fatty acid to amine is from 0.60 to 1 mol of fatty acid per 1 mol of hydroxyl group of the alkanolamine, preferably from 0.80 to 1 mol of fatty acid per 1 mol of hydroxyl group of the alkanolamine.

In an embodiment of the present invention, component (a) comprises a mixture of one or more mono-esteramine, di-esteramine and/or tri-esteramine compounds of formula (111), (II2), and (II3), respectively, wherein m=n=p; R₁-C(O)- is a linear acyl group wherein R₁ is an alkyl or alkenyl group containing from 11 to 21 carbon atoms, preferably derived from hydrogenated or non-hydrogenated tallow fatty acid; R₂ and R₃ each represent -OH; q is 0 (i.e. the compound is not ethoxylated). Such compound may be produced by esterifying tallow fatty acid and triethanolamine, wherein the ratio of tallow fatty acid to amine is 1 mol of tallow fatty acid per 1 mol of hydroxyl group of the triethanolamine.

In another embodiment of the present invention, component (a) comprises a mixture of one or more mono-esteramine, di-esteramine and/or tri-esteramine compounds of formula (111), (II2), and (II3), respectively, wherein m=p, n=1, R₁-C(O)- is a linear acyl group wherein R₁ is a linear alkyl or alkenyl containing from 11 to 21 carbon atoms, preferably derived from hydrogenated or non-hydrogenated tallow fatty acid; R₂ represents -H, R₃ represents -OH, q is 0 (i.e. the compound is not ethoxylated). Such compound may be produced by esterifying tallow fatty acid and methyldiethanolamine, wherein the ratio of tallow fatty acid to amine is 1 mol of tallow fatty acid per 1 mol of hydroxyl group of the methyldiethanolamine.

The product resulting from the esterification reaction comprises a mixture of mono-, di- and tri-esters of fatty acids. The product may also contain free alkanolamine and free fatty acid. The progress of the reaction may be monitored using conventional techniques, e.g. TLC, GC or HPLC. The reaction may, for example, be monitored by consumption of the fatty acid.

### Component (b): Solvent

The present invention comprises a component (b), said component comprising at least a solvent, wherein the solvent is selected from mineral oils, paraffins and waxes derived from petroleum, and vegetal or animal fats and oils, and wherein component (b) content is 50% wt. or more, based on the total weight of the composition.

In another embodiment of the invention, preferred solvents are mineral oils, specially preferred are paraffin oils or mixtures of paraffin oils with macrocrystalline paraffins and microcrystalline paraffins; and vegetal or animal fats and oils, such as rapeseed oil, palm oil or tallow.

### MINERAL OILS, PARAFFINS AND WAXES DERIVED FROM PETROLEUM:

Suitable mineral oils, as well as paraffins and waxes derived from petroleum according to the present invention are:
- aromatic oils, which are a mixture of mineral oils from petroleum with a high content of components having aromatic type rings,
- white mineral oils, which are highly refined petroleum derivatives, generally used as carriers, excipients and lubricants in different industrial applications,
- paraffin oils, which are petroleum derivatives rich in paraffin components and have low density and a variable viscosity,
- macrocrystalline paraffins, which are petroleum derivatives containing mainly linear carbon chains with a molecular weight comprised between 250 and 500 and, although they are solids at room temperature, they have low melting points, usually comprised between 40°C and 70°C,
- microcrystalline paraffins, which are petroleum derivatives and are mainly saturated hydrocarbons in which linear chains with short branches (isoparaffins) are predominant. They usually have mean molecular weights comprised between 500 and 800, and are solids at room temperature, having melting points comprised between 70°C and 100°C.

### ANIMAL OR VEGETABLE FATS AND OILS

Suitable animal or vegetable fats and oils according to the present invention are esters of linear and/or branched, saturated and/or unsaturated alkanecarboxylic acids with a chain length of 1 up to 30 carbon atoms and linear and/or branched, saturated and/or unsaturated alcohols with a chain length of 1 up to 30 carbon atoms, of the group of esters of aromatic carboxylic acids and linear and/or branched, saturated and/or unsaturated alcohols with a chain length of 1 up to 30 carbon atoms. These oils can be advantageously selected from the group consisting of isopropyl myristate, isopropyl palmitate, isopropyl stearate, isopropyl oleate, n-butyl stearate, n-hexyl laurate, n-decyl oleate, isooctyl stearate, isononyl stearate, isononyl isononanoate, 2-ethylhexyl laurate, 2-ethylhexyl palmitate, 2-ethylhexyl cocoate, 2-hexyldecyl stearate, 2-ethylhexyl isostearate, 2-octyldodecyl palmitate, cetyl palmitate, oleyl oleate, oleyl erucate, erucyl oleate, erucyl erucate, as well as synthetic, semisynthetic and natural mixtures of such esters, such as jojoba oil (a natural mixture of esters of monounsaturated monocarboxylic acids with a C18-C24 chain with also monounsaturated monoalcohols and with a long C18-C24 chain). Other suitable oils of the type of esters of saturated alkanecarboxylic acids and alcohols are fatty acid methyl esters, preferably C6-C24 fatty acid methyl esters from animal and vegetable fats and oils such as cotton, safflower, coconut, rapeseed, linseed, palm, palm kernel, sunflower, olein, olive, olive pomace, castor oil, tallow, soy, tall oil, etc, possibly totally or partially hydrogenated, as well as purified or synthetic fatty acids such as caproic acid, caprylic acid, capric acid, lauric acid, myristic acid, palmitic acid, palmitoleic acid, stearic acid, isostearic acid, 2-ethylhexanoic acid, oleic acid, ricinoleic acid, elaidic acid, petroselinic acid, linoleic acid, linolenic acid, arachidic acid, gadoleic acid, behenic acid and erucic acid, or mixtures thereof.

Other suitable animal or vegetable fats and oils according to the present invention are fatty acid triglycerides, specifically triglycerin esters of linear and/or branched, saturated and/or unsaturated alkanecarboxylic acids with a chain length of 6 up to 24 carbon atoms, preferably of 10 up to 18 carbon atoms. The fatty acids esterifying the different positions of glycerin can be different, giving rise to a large amount of possible combinations, including positional combinations. The position of the different fatty acids in natural triglycerides is not random, but rather it depends on the origin of the fat. The triglycerides more simple are those constituted by a sole fatty acid.

Fatty acid triglycerides can be selected, for example, from the group consisting of synthetic, semi-synthetic and natural oils, as for example, animal fats and oils such as cow tallow, pig lard, bone oil, aquatic animal fats and oils (fish, such as herring, cod or sardine; cetaceans; etc.); and vegetable fats and oils such as avocado oil, almond oil, hazelnut oil, babassu palm oil, borage oil, peanut oil, canola oil, hemp oil, milk thistle oil, safflower oil, chufa oil, coconut oil, rapeseed oil, black cumin oil, wheat germ oil, sunflower oil, linseed oil, macadamia nut oil, corn oil, walnut oil, olive oil and its byproducts such as olive pomace oil, palm oil and its fractions such as palm olein and palm stearin, evening primrose oil, rosehip oil, castor oil, rice bran oil, apricot kernel oil, cottonseed oil, pumpkinseed oil, palm kernel oil and its fractions such as palm kernel olein and palm kernel stearin, grape seed oil, sesame oil, soy oil, cocoa butter, shea butter and the like.

From the environmental point of view, animal and vegetable fats and oils are preferred as solvent, since the use of petroleum derivatives is thus avoided. Although for the person skilled in the art it is evident that, alternatively, animal and vegetable fats and oils can also be combined with mineral oils, paraffins and waxes from petroleum, such that the resulting solvent has suitable properties from the environmental point of view

### (biodegradability, ecotoxicity, etc.)

The composition according to the present invention may contain further components.

In a preferred embodiment of the invention, the composition comprises:
- a component (a) comprising a mixture of one or more mono-esteramine, di-esteramine and/or tri-esteramine compounds of formula (II1), (II2), and (II3), respectively, wherein in said component the sum weight percentage of di-esteramine and tri-esteramine compounds ((II2) and (II3)) is from 70 to 100% wt., based on the total weight of esterified species comprised in component (a).
- a component (b) comprising at least a solvent, wherein the solvent is selected from mineral oils, paraffins and waxes derived from petroleum, and vegetal or animal fats and oils, and wherein component (b) content is 50% wt. or more, based on the total weight of the composition.

Preferably, the above defined compositions are intended for preventing the caking of solid fertilizers.

### ANTICAKING COMPOSITIONS OF THE INVENTION

As indicated above, the composition of the present invention comprises:
- a component (a) comprising a mixture of one or more mono-esteramine, di-esteramine and/or tri-esteramine compounds of formula (II1), (II2), and (II3), respectively, wherein in said component the sum weight percentage of di-esteramine and tri-esteramine compounds ((II2) and (II3)) is from 70 to 100% wt., based on the total weight of esterified species comprised in component (a);
- a component (b) comprising at least a solvent, wherein the solvent is selected from mineral oils, paraffins and waxes derived from petroleum and vegetal and animal fats and oils, and wherein component (b) content is 50% wt. or more, based on the total weight of the composition.

In an embodiment of the present invention, the component (a) comprises a mixture of one or more mono-esteramine, di-esteramine and/or tri-esteramine compounds of formula (II1), (II2), and (II3), respectively, which results from the esterification process.

In an embodiment of the present invention, the component (a) comprises a mixture of one or more mono-esteramine, di-esteramine and/or tri-esteramine compounds of formula (II1), (II2), and (II3), respectively.

In another embodiment of the invention, the composition comprises:
- a component (a) comprising a mixture of one or more mono-esteramine, di-esteramine and/or tri-esteramine compounds of formula (II1), (II2), and (II3), respectively, wherein in said component the sum weight percentage of di-esteramine and tri-esteramine compounds ((II2) and (II3)) is from 70 to 100% wt., based on the total weight of esterified species comprised in component (a);
- a component (b) comprising at least a solvent, wherein the solvent is selected from mineral oils, paraffins and waxes derived from petroleum, and vegetal or animal fats and oils, and wherein component (b) content is 50% wt. or more, based on the total weight of the composition;
wherein the amount of water of the composition is 1% wt. or less, preferably 0.5% wt. or less.

In a preferred embodiment, the composition of the present invention consists of:
- a component (a) comprising a mixture of one or more mono-esteramine, di-esteramine and/or tri-esteramine compounds of formula (II1), (II2), and (II3), respectively, wherein in said component the sum weight percentage of di-esteramine and tri-esteramine compounds ((II2) and (II3)) is from 70 to 100% wt., based on the total weight of esterified species comprised in component (a), preferably from 75 to 100% wt. and more preferably from 80 to 100% wt.;
- a component (b) comprising at least a solvent, wherein the solvent is selected from mineral oils, paraffins and waxes derived from petroleum, and vegetal or animal fats and oils, and wherein component (b) content is 50% wt. or more, based on the total weight of the composition.

In another preferred embodiment, the composition of the present invention consists of:
- a component (a) comprising a mixture of one or more mono-esteramine, di-esteramine and/or tri-esteramine compounds of formula (II1), (II2), and (II3), respectively, wherein in said component the weight percentage of tri-esteramine compound (II3) is 100% wt., based on the total weight of esterified species comprised in component (a);
- a component (b) comprising at least a solvent, wherein the solvent is selected from mineral oils, paraffins and waxes derived from petroleum, and vegetal or animal fats and oils, and wherein component (b) content is 50% wt. or more, based on the total weight of the composition.

In another preferred embodiment, the composition of the present invention consists of:
- a component (a) comprising a mixture of one or more mono-esteramine, di-esteramine and/or tri-esteramine compounds of formula (II1), (II2), and (II3), respectively, wherein in said component the weight percentage of tri-esteramine compound (II3) is 100% wt., based on the total weight of esterified species comprised in component (a);
- a component (b) comprising at least a solvent, wherein the solvent is selected from mineral oils, paraffins and waxes derived from petroleum, and vegetal or animal fats and oils, and wherein component (b) content is 50% wt. or more, based on the total weight of the composition;
wherein the amount of water of the composition is 1% wt. or less, preferably 0.5% wt. or less.

In another preferred embodiment, component (a) comprises a mixture of one or more mono-esteramine, di-esteramine and/or tri-esteramine compounds of formula (II1), (II2), and (II3), respectively, wherein m=n=p=2; R₁-C(O)- is a linear acyl group wherein R₁ is a linear alkyl or alkenyl containing from 11 to 21 carbon atoms, preferably derived from hydrogenated or non-hydrogenated tallow fatty acid; R₂ and R₃ each represent -OH, and q is 0 (i.e. the compound is not alkoxylated). Such a compound may be produced by esterifying tallow fatty acid and triethanolamine, wherein the ratio of tallow fatty acid to amine is 1 mol of tallow fatty acid per 1 mol of hydroxyl group of the triethanolamine. This mixture is preferably combined with a solvent selected from vegetal and animal fats and oils. However, it can also be combined with mineral oils, paraffins and waxes derived from petroleum.

The compositions according to the invention for preventing caking of solid fertilizers preferably comprise the individual components in the following amounts, expressed as percentage by weight, with respect to the total weight of the composition:
- 5% to 50% of a mixture of one or more mono-esteramine, di-esteramine and/or tri-esteramine compounds of component (a),
- 50% to 95% of a solvent of component (b).

Preferably, a mixture of one or more mono-esteramine, di-esteramine or tri-esteramine compounds of component (a) is contained in an amount, expressed as percentage by weight, of 10% to 30%, more preferably of 15% to 25%.

Preferably, a solvent of component (b) is contained in an amount, expressed as percentage by weight, of 55% to 90%, more preferably of 65% to 85%.

In a preferred embodiment of the invention, component (b) comprises mineral oils, paraffins, waxes from petroleum, or mixtures thereof.

In another preferred embodiment of the invention, the composition comprises component (b) comprising animal and vegetable fats and oils.

In another embodiment of the invention, the composition comprises, in the indicated amounts expressed as percentage by weight with respect to the total weight of the composition:
- 5% to 50% of a mixture of one or more mono-esteramine, di-esteramine and/or tri-esteramine compounds of component (a),
- 50% to 95% of a solvent of component b),
wherein the amount of water in the composition is 1% wt. or less, preferably 0.5% wt. or less.

In another preferred embodiment of the invention, in the composition of the invention comprising components (a) and (b), component (b) comprises mineral oils, paraffins and/or waxes from petroleum.

In another embodiment of the invention, preferred composition is that comprising component (b) that comprises animal and vegetable fats and oils.

The compositions of the present invention can be obtained with a conventional process for mixing the different components, well known by the skilled person. For example, the different components can be mixed in molten state and once the mixture has been homogenized, it is packaged and possibly cooled.

The compositions object of the present invention have, in relation to solid fertilizers, a broad field of applications, since they are effective both for granular simple and granular complex fertilizers. They are suitable for granular simple and granular complex fertilizers of the N, NP, NK and NPK type, being especially preferable for granular complex fertilizers of the NPK and AN type.

The use of compositions object of this invention in solid fertilizers provides said fertilizers with an efficient resistance against caking, making the fertilizers show excellent properties regarding their breakdown, even after their transport and storage period. Said use also forms part of the invention.

Another object of the invention is the process for applying the anticaking compositions. These processes are well known by the skilled person and consist, for example, but not limited to, in applying the compositions according to the invention, in molten state, by means of spraying technique, on the surface of the solid fertilizer, once such fertilizer has been manufactured.

The amount of additive to be applied on the surface of the fertilizer will depend in each case on the humidity, storage time and temperature conditions expected for the storage of the fertilizer, although good results can be obtained when said compositions are applied on the fertilizer in proportions comprised between 500 and 5000 ppm with respect to the weight of the fertilizer, preferably between 700 and 3000 pm.

Fertilizer compositions, wherein solid fertilizers are coated by applying on the surface a composition according to the invention are also a part of the invention. A composition according to the invention can be applied to both types of fertilizers, granular simple fertilizers and granular complex fertilizers, being especially preferable for granular complex fertilizers of the NPK type and for ammonium nitrate type.

The following examples are given in order to provide a person skilled in the art with a sufficiently clear and complete explanation of the present invention, but should not be considered as limiting of the essential aspects of its subject, as set out in the preceding portions of this description.

### EXAMPLES

The first part of the Examples section refers to the preparation of the anticaking compositions according to the present invention.

The second part of the Examples section refers to the performance of anticaking effect of the compositions according to the present invention.

### EXAMPLE 1

### Preparation of the component a (a1 to a4)

### Preparation of component a1

606,6 of hydrogenated tallow acid were introduced in an inert atmosphere into a reactor, and 109,6g of triethanolamine were added with stirring. The mixture was heated for at least 4 hours at 160-180°C in order to remove the water of the reaction. The progress of the reaction was monitored by an acid value assay, which determines the residual acidity, to get an esterification of at least 90 to 95% of the fatty acids, and 669,1g of product were obtained.

The composition of the product is determined with 1H Nuclear Magnetic Ressonance (1H-NMR) at 500 MHz, using pyridine as solvent and TMS as reference. The percentages by weight of di-esteramine and tri-esteramine compounds measured in component a1 are normalized on the basis of 100%.

The sum weight percentage of di-esteramine and tri-esteramine compounds is 98.6%.

### Preparation of components a2 to a4

All other components (a2 to a4) used to prepare the anticaking compositions of Table 1 were prepared analogously to a1.

Table 1 summarizes the sum weight percentages of di-esteramine and tri-esteramine compounds and the ratio of mol of fatty acid per mol of hydroxyl group of the alkanolamine of the prepared components a1 to a4:

**TABLE 1**

| Component (a) | Fatty acid | Alkanolamine | % Sum weight of di-esteramine + tri-esteramine | Ratio mol COOH: mol OH of alkanolamine |
|---|---|---|---|---|
| a1 | Hydrogenated tallow | Triethanolam ine | 98.6 | 1:1 |
| a2 | Stearic | Triethanolam ine | 98.1 | 1:1 |
| a3 | Hydrogenated tallow | Methyldietha nolamine | 99.4 | 1:1 |
| a4 | Stearic | Methyldietha nolamine | 99.1 | 1:1 |

### EXAMPLES 2 TO 9

### Preparation of anticaking compositions A, B, C, D, E, F, G, H

Compositions A, B, C, D, E, F, G, H according to the present invention are detailed in Table 2. The amounts of the indicated components are expressed as parts by weight of each component added to each composition, and were prepared according to the following general method:
The solvent is loaded in a vessel provided with stirring at the temperature necessary for achieving a good flowability in the end product, maximum 90°C. The stirring is maintained and the corresponding component a (a1, a2, a3, a4) is added, maintaining the same temperature. The mixture is left under stirring for 30 minutes at the same temperature. Unloading and packaging are subsequently performed.

**TABLE 2**

| Anti-caking compositions | | | | | | | | |
|---|---|---|---|---|---|---|---|---|
| Components | A | B | C | D | E | F | G | H |
| a1 | 20,0 | --- | --- | --- | 15,0 | --- | --- | --- |
| a2 | --- | 20,0 | --- | --- | --- | 15,0 | --- | --- |
| a3 | --- | --- | 20,0 | --- | --- | --- | 15,0 | --- |
| a4 | --- | --- | --- | 20,0 | --- | --- | --- | 15,0 |
| Paraffinic process oil SN-100 | 80,0 | 80,0 | 80,0 | 80,0 | --- | --- | --- | --- |
| Stearic acid (Edenor C18 98-100, Cognis) | --- | --- | --- | --- | 10,0 | 10,0 | 10,0 | 10,0 |
| Crude rapeseed oil (Gustav Heess) | --- | --- | --- | --- | 75,0 | 75,0 | 75,0 | 75,0 |

### EXAMPLE 10

### Anticaking activity evaluation method: Accelerated Caking Test

Portions of a granulated fertilizer (NPK 21-3P-8K) from Borealis were treated with compositions A, B, C, D, E, F, G, H detailed in Table 2, and also with paraffinic process oil SN-100 and crude rapeseed oil on their own as references, by means of spraying said molten compositions and reference materials on the fertilizer, in a rotary mixer, at a dose of 1500 ppm with respect to the weight of the fertilizer (1.5 Kg/MT).

Respective representative samples of fertilizer treated with the anti-caking compositions according to the invention and samples of fertilizer treated with the reference materials and sample of untreated fertilizer were subjected to an accelerated caking test. The test consisted of the following steps: First, 90 g of fertilizer were introduced in cylindrical perforated metal test tubes with a height of 60 mm and a diameter of 45 mm, which can be longitudinally opened for extracting the samples, 2 test tubes being used for each sample of fertilizer. Once the samples had been introduced in the tubes, they were subjected to a pressure of 1.26 Kg/cm² in an INSTRON dynamometer, model 1011. The test tubes were maintained, at the previously indicated pressure, at a relative humidity (R.H.) of 70% and at a temperature of 20°C for a period of 24 hours in a HERAEUS climatic chamber, model HC 2057. The climatic conditions were subsequently changed, passing to a R.H. of 20% and 40°C for a period of 2 days.

Finally, the test tubes were left at environmental temperature and humidity.

The first aspect that was evaluated is the agglomeration of the fertilizer. The agglomeration average in percentage is a measure of the extension of the agglomeration experimented by the sample. The second measure is the resistance to break up. The latter is measured in the already indicated dynamometer, at a rate of 10 mm/minute. The obtained result is expressed as the average resistance to break up in kg.

The results are shown in Table 3.

**TABLE 3**

| Results of the caking tests | | |
|---|---|---|
| Additive (Dosage: 1.5 kg/MT) | Average % of agglomeration before break up | Average resistance to break up (kg) |
| A | 100,0 | 18,4 |
| B | 100,0 | 22,8 |
| C | 100,0 | 35,5 |
| D | 100,0 | 35,7 |
| Paraffinic process oil SN-100 | 100,0 | 43,4 |
| E | 100,0 | 26,0 |
| F | 100,0 | 29,4 |
| G | 100,0 | 32,0 |
| H | 100,0 | 34,9 |
| Crude rapeseed oil | 100,0 | 40,5 |
| Untreated blank | 100,0 | 107,5 |

### EXAMPLE 11

### Anticaking activity evaluation method: Accelerated Caking Test

Other compositions according to the present invention were tested following the method described in Example 10, this time with a granulated fertilizer (NPK 20-8-8) from Borealis at a dose of 1500 ppm with respect to the weight of the fertilizer (1.5 Kg/MT).

The test tubes were maintained at the pressure indicated in Example 10, at a relative humidity (R.H.) of 60% and at a temperature of 20°C for a period of 63 hours in a HERAEUS climatic chamber, model HC 2057. The climatic conditions were subsequently changed, passing to a R.H. of 20% and 40°C for a period of 2 days.

Some compositions described in Examples 2 to 9 were tested, together with paraffinic process oil SN-100 and crude rapeseed oil on their own as references. The obtained result is expressed as the agglomeration average in percentage and if the agglomeration has been complete, also as the average resistance to break up in kg.

The obtained results are shown in Table 4.

**TABLE 4**

| Results of the caking tests | | |
|---|---|---|
| Additive (Dosage: 1.5 kg/MT) | Average % of agglomeration before break up | Average resistance to break up (kg) |
| A | 87,3 | --- |
| B | 100,0 | 10,9 |
| C | 100,0 | 15,6 |
| D | 100,0 | 21,2 |
| Paraffinic process oil SN-100 | 100,0 | 51,5 |
| F | 100,0 | 22,1 |
| H | 100,0 | 35,1 |
| Crude rapeseed oil | 100,0 | 56,4 |
| Untreated blank | 100,0 | 173,8 |

From the obtained results, it is deduced, as in Example 10, that some compositions of the invention provide good results as anti-caking agents comparing to the blank and the references. Compositions A and B are especially preferred for these two fertilizers.

### EXAMPLE 12

### Anticaking activity evaluation method: Accelerated Caking Test

Other compositions according to the present invention were tested following the method described in Example 10, this time with Ammonium Sulfonitrate (ASN) from Borealis at a dose of 1500 ppm with respect to the weight of the fertilizer (1.5 Kg/MT). The test tubes were maintained, at the pressure indicated in Example 2, at a relative humidity (R.H.) of 70% and at a temperature of 20°C for a period of 15 hours in a HERAEUS climatic chamber, model HC 2057. The climatic conditions were subsequently changed, passing to a R.H. of 20% and 40°C for a period of 2 days. Some compositions already described in Examples 2 to 9 were tested, together with crude rapeseed oil on its own as a reference.

The obtained result is expressed as the agglomeration average in percentage and if the agglomeration has been complete, also as the average resistance to break up in kg. The obtained results are shown in Table 5.

**TABLE 5**

| Results of the caking tests | | |
|---|---|---|
| Additive (Dosage: 1.5 kg/MT) | Average % of agglomeration before break up | Average resistance to break up (kg) |
| E | 100,0 | 3,2 |
| F | 62,8 | --- |
| G | 100,0 | 6,9 |
| H | 100,0 | 9,8 |
| Crude rapeseed oil | 100,0 | 24,6 |
| Untreated blank | 100,0 | 46,8 |

From the obtained results, it can be seen that the compositions of the present invention can be used as anti-caking agents for solid fertilizers, providing good results in their performance.

## Claims

1. A composition for preventing the caking of solid fertilizers comprising:
- a component (a) which comprises a mixture of one or more mono-esteramine, di-esteramine and/or tri-esteramine of formula (II1), (II2), and (II3), respectively: 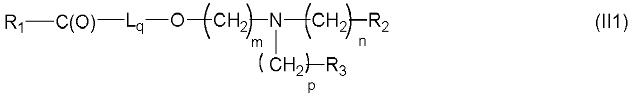 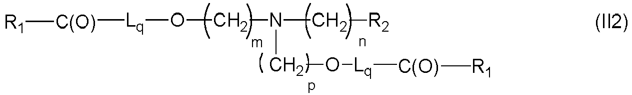 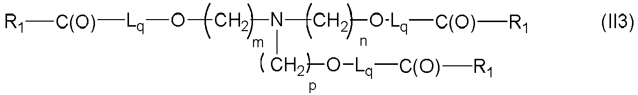 wherein in formula II1, II2, and II3
R₁ is a linear or branched alkyl containing 5 to 23 carbon atoms and/or a linear alkenyl group containing 5 to 23 carbon atoms and from 1 to 3 double bonds,
R₂ and R₃ each independently represent -H or-OH,
L represents a -(OCH₂CH₂)ₐ-(OCHR₄CH₂)_{b}- group, wherein R₄ represents an alkyl group containing 1 to 4 carbon atoms, a represents a number within the range of 0 to 20, b represents a number within the range of 0 to 6, and the sum of a+b represents the average alkoxylation degree which corresponds to a number from 0 to 26,
m, n, p each independently represent a number within the range from 1 to 4, and q represents a number with the range from 0 to 26;
wherein the sum weight percentage of di-esteramine and tri-esteramine compounds ((II2) and (II3)) is from 70 to 100% wt., based on the total weight of esterified species comprised in component (a);
- a component (b) which comprises at least a solvent, wherein the solvent is selected from mineral oils, paraffins and waxes derived from petroleum, and vegetal or animal fats and oils, or mixtures thereof, and wherein component (b) content is 50% wt. or more, based on the total weight of the composition.

2. The composition according to claim 1, **characterized in that** in formula (II1) and (II2), R₂ and R₃ independently represent -OH.

3. The composition according to any one of claims 1 to 2, **characterized in that** in formula (II1), (II2), and (II3), m, n and p represent number 2.

4. The composition according to any one of claims 1 to 3, **characterized in that** in formula (II1), (II2), and (II3), R₁ independently represents a linear or branched alkyl or a linear alkenyl group containing 11 to 21 carbon atoms.

5. The composition according to any one of claims 1 to 4, **characterized in that** the weight percentage of the tri-esteramine compound (II3) is 100% wt., based on the total weight of esterified species comprised in component (a).

6. The composition according to any one of claims 1 to 5, **characterized in that** component (b) is selected from mineral oils and paraffins.

7. The composition according to any one of claims 1 to 6, **characterized in that** component (b) comprises a mixture of one or more fatty acids selected from tallow, hydrogenated tallow, and stearic acid, wherein the mixture content is up to 30% wt., based on the total weight of the composition.

8. The composition according to any one of claims 1 to 7, **characterized in that** the components are comprised in the following amounts, expressed as weight percentage based on the total weight of the composition:
- 5% to 50% of component (a),
- 50% to 95% of component (b).

9. Use of a composition according to any one of claims 1 to 8 for preventing the caking of solid fertilizers.

10. A method for preparing a composition according to any one of claims 1 to 8,said method comprising i) preparing component (a) by esterification, reacting a fatty acid or a mixture of fatty acids with an alkanolamine or a mixture of alkanolamines, and ii) adding component (b).

11. Solid fertilizers, which particles have been coated or mixed with a composition according to any one of claims 1 to 8.

12. A method for preparing solid fertilizers resistant to caking, **characterized in that** the solid fertilizer particles are coated or mixed with a composition according to any one of claims 1 to 8.

## Patentansprüche

1. Antiklumpzusammensetzung für feste Düngemittel, umfassend:
- Komponente (a), umfassend eine Mischung eines oder mehrerer Monoesteramine, Diesteramine und/oder Triesteramine der Formel (II1), (II2) bzw. (II3): 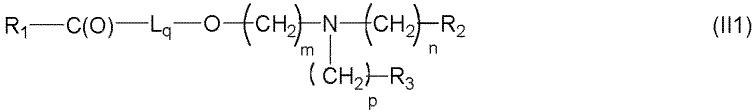 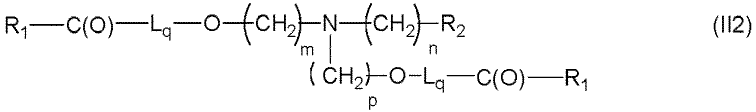 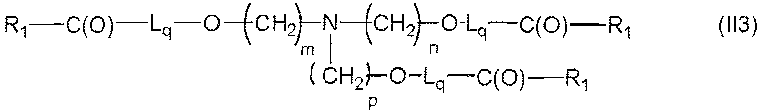 wobei in Formel II1, II2 und II3
R₁ eine lineare oder verzweigte Alkylgruppe, enthaltend 5 bis 23 Kohlenstoffatome, und/oder eine lineare Alkenylgruppe, enthaltend 5 bis 23 Kohlenstoffatome und 1 bis 3 Doppelbindungen, ist,
R₂ und R₃ jeweils unabhängig -H oder -OH darstellen,
L eine -(OCH₂CH₂)ₐ-(OCHR₄CH₂)_{b}-Gruppe darstellt, wobei R₄ eine Alkylgruppe darstellt, die 1 bis 4 Kohlenstoffatome enthält, a eine Zahl im Bereich von 0 bis 20 darstellt, b eine Zahl im Bereich von 0 bis 6 darstellt und die Summe von a+b den durchschnittlichen Alkoxylierungsgrad darstellt, der einer Zahl von 0 bis 26 entspricht,
m, n, p jeweils unabhängig eine Zahl im Bereich von 1 bis 4 darstellen und q eine Zahl im Bereich von 0 bis 26 darstellt;
wobei die Summe der Gewichtsanteile der Diesteramin- und Triesteraminverbindungen ((II2) und (II3)) 70 bis 100 Gew.-% beträgt, basierend auf dem Gesamtgewicht an in Komponente (a) enthaltenen veresterten Spezies;
- Komponente (b), umfassend mindestens ein Lösungsmittel, wobei das Lösungsmittel aus Mineralölen, Paraffinen und Wachsen, die von Erdöl abgeleitet sind, und pflanzlichen oder tierischen Fetten und Ölen oder Mischungen davon ausgewählt ist und wobei der Gehalt an Komponente (b) 50 Gew.-% oder mehr beträgt, basierend auf dem Gesamtgewicht der Zusammensetzung.

2. Zusammensetzung gemäß Anspruch 1, **dadurch gekennzeichnet, dass** in Formel (II1) und (II2) R₂ und R₃ jeweils unabhängig -OH darstellen.

3. Zusammensetzung gemäß einem der Ansprüche 1 bis 2, **dadurch gekennzeichnet, dass** in Formel (II1), (II2) und (II3), m, n und p 2 darstellen.

4. Zusammensetzung gemäß einem der Ansprüche 1 bis 3, **dadurch gekennzeichnet, dass** in Formel (II1), (II2) und (II3) R₁ unabhängig eine lineare oder verzweigte Alkylgruppe oder eine lineare Alkenylgruppe, enthaltend 11 bis 21 Kohlenstoffatome, darstellt.

5. Zusammensetzung gemäß einem der Ansprüche 1 bis 4, **dadurch gekennzeichnet, dass** der Gewichtsanteil der Triesteraminverbindung (II3) 100 Gew.-% beträgt, basierend auf dem Gesamtgewicht an in Komponente (a) enthaltenen veresterten Spezies.

6. Zusammensetzung gemäß einem der Ansprüche 1 bis 5, **dadurch gekennzeichnet, dass** Komponente (b) aus Mineralölen und Paraffinen ausgewählt ist.

7. Zusammensetzung gemäß einem der Ansprüche 1 bis 6, **dadurch gekennzeichnet, dass** Komponente (b) eine Mischung einer oder mehrerer Fettsäuren umfasst, ausgewählt aus Talgfettsäure, hydrogenierter Talgfettsäure und Stearinsäure, wobei der Gehalt dieser Mischung bis zu 30 Gew.-% beträgt, basierend auf dem Gesamtgewicht der Zusammensetzung.

8. Zusammensetzung gemäß einem der Ansprüche 1 bis 7, **dadurch gekennzeichnet, dass** die Komponenten in den folgenden Mengen enthalten sind, ausgedrückt als Gewichtsprozent, basierend auf dem Gesamtgewicht der Zusammensetzung:
- 5 % bis 50 % der Komponente (a);
- 50 % bis 95 % der Komponente (b).

9. Verwendung einer Zusammensetzung gemäß einem der Ansprüche 1 bis 8 zur Verhinderung von Klumpenbildung fester Düngemittel.

10. Verfahren zum Herstellen einer Zusammensetzung gemäß einem der Ansprüche 1 bis 8, wobei das Verfahren umfasst i) Herstellen von Komponente (a) mittels Veresterung, Reagierenlassen einer Fettsäure oder einer Mischung von Fettsäuren mit einem Alkanolamin oder einer Mischung von Alkanolaminen, und ii) Zugeben von Komponente (b).

11. Feste Düngemittel, deren Partikel mit einer Zusammensetzung gemäß einem der Ansprüche 1 bis 8 beschichtet oder vermischt worden sind.

12. Verfahren zum Herstellen fester Düngemittel, die gegen Klumpenbildung resistent sind, **dadurch gekennzeichnet, dass** die festen Düngemittelpartikel mit einer Zusammensetzung gemäß einem der Ansprüche 1 bis 8 beschichtet oder vermischt sind.

## Revendications

1. Composition pour empêcher l'agglomération d'engrais solides comprenant :
- un composant (a) qui comprend un mélange d'une ou de plusieurs mono-esteramines, di-esteramines et/ou tri-esteramines de formules (II1), (II2) et (II3), respectivement : 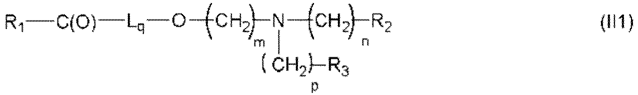 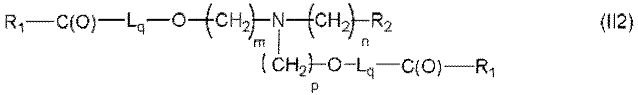 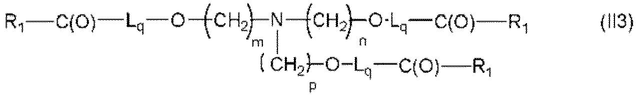 dans laquelle dans les formules II1, II2 et II3
R₁ est un alkyle linéaire ou ramifié contenant de 5 à 23 atomes de carbone et/ou un groupe alcényle linéaire contenant de 5 à 23 atomes de carbone et de 1 à 3 doubles liaisons,
R₂ et R₃ représentent chacun indépendamment -H ou -OH,
L représente un groupe -(OCH₂CH₂)ₐ-(OCHR₄CH₂)_{b}-, dans laquelle R₄ représente un groupe alkyle contenant 1 à 4 atomes de carbone, a représente un nombre dans la plage de 0 à 20, b représente un nombre dans la plage de 0 à 6, et la somme de a+b représente le degré moyen d'alcoxylation qui correspond à un nombre de 0 à 26,
m, n, p représentent chacun indépendamment un nombre dans la plage de 1 à 4, et q représente un nombre dans la plage de 0 à 26 ;
dans laquelle le pourcentage en poids total des composés de di-esteramine et de tri-esteramine ((II2) et (II3)) est de 70 à 100% en poids, sur la base du poids total des espèces estérifiées comprises dans le composant (a) ;
- un composant (b) qui comprend au moins un solvant, dans laquelle le solvant est sélectionné parmi les huiles minérales, les paraffines et les cires dérivées du pétrole, et les graisses et huiles végétales ou animales, ou les mélanges de celles-ci, et dans laquelle la teneur en composant (b) est de 50 % en poids ou plus, sur la base du poids total de la composition.

2. Composition selon la revendication 1, **caractérisée en ce que** dans les formules (II1) et (II2), R₂ et R₃ représentent indépendamment -OH.

3. Composition selon l'une quelconque des revendications 1 à 2, **caractérisée en ce que** dans les formules (II1), (II2) et (II3), m, n et p représentent le nombre 2.

4. Composition selon l'une quelconque des revendications 1 à 3, **caractérisée en ce que** dans les formules (II1), (II2) et (II3), R₁ représente indépendamment un alkyle linéaire ou ramifié ou un groupe alcényle linéaire contenant de 11 à 21 atomes de carbone.

5. Composition selon l'une quelconque des revendications 1 à 4, **caractérisée en ce que** le pourcentage en poids du composé de tri-esteramine (II3) est de 100 % en poids, sur la base du poids total des espèces estérifiées comprises dans le composant (a).

6. Composition selon l'une quelconque des revendications 1 à 5, **caractérisée en ce que** le composant (b) est sélectionné parmi les huiles minérales et les paraffines.

7. Composition selon l'une quelconque des revendications 1 à 6, **caractérisée en ce que** le composant (b) comprend un mélange d'un ou de plusieurs acides gras sélectionnés parmi un suif, un suif hydrogéné et l'acide stéarique, dans laquelle la teneur en mélange va jusqu'à 30 % en poids, sur la base du poids total de la composition.

8. Composition selon l'une quelconque des revendications 1 à 7, **caractérisée en ce que** les composants sont compris dans les quantités suivantes, exprimées en pourcentage en poids sur la base du poids total de la composition :
- 5 % à 50 % de composant (a),
- 50 % à 95 % de composant (b).

9. Utilisation d'une composition selon l'une quelconque des revendications 1 à 8 pour empêcher l'agglomération d'engrais solides.

10. Procédé de préparation d'une composition selon l'une quelconque des revendications 1 à 8, ledit procédé comprenant i) la préparation du composant (a) par estérification, la réaction d'un acide gras ou d'un mélange d'acides gras avec une alcanolamine ou un mélange d'alcanolamines, et ii) l'ajout du composant (b).

11. Engrais solides, lesquelles particules ont été enrobées ou mélangées avec une composition selon l'une quelconque des revendications 1 à 8.

12. Procédé de préparation d'engrais solides résistant à l'agglomération, **caractérisé en ce que** les particules d'engrais solides sont enrobées ou mélangées avec une composition selon l'une quelconque des revendications 1 à 8.
